# FASCICULE DE BREVET EUROPEEN

(11) **EP 1 061 895 B1**
(45) Date de publication et mention de la délivrance du brevet: **23.04.2003**
(21) Numéro de dépôt: 99907660.7
(22) Date de dépôt: 04.03.1999
(51) Int. Cl.: A61K 7/48

(54) **COMPOSITION SYNERGIQUE COMPRENANT UN COMPOSE A STRUCTURE LIPOAMINOACIDE ET UN EXTRAIT DE NENUPHAR**
SYNERGISTISCHE ZUSAMMENSETZUNG ENTHALTEND EINER LIPOAMINOSÄURE STRUKTUR UND EIN NENUPHAR-EXTRAKTE
SYNERGETIC COMPOSITION COMPRISING A COMPOUND WITH LIPOAMINOACID STRUCTURE AND A TUBEROUS WATER-LILY EXTRACT

(30) Priorité: 09.03.1998 FR 9802828
(43) Date de publication de la demande: 27.12.2000
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: STOLTZ, Corinne, 94320 THIAIS (FR)
(74) Mandataire: Conan, Philippe Claude
(86) Numéro de dépôt international: FR9900493
(87) Numéro de publication internationale: WO99045899

(56) Documents cités:
- WO-A-92/21318
- CHEMICAL ABSTRACTS, vol. 111, no. 12, 18 septembre 1989 (1989-09-18) Columbus, Ohio, US; abstract no. 102551, XP002089107 & JP 01 047708 A (SHISEIDO CO.) 22 février 1989 (1989-02-22)
- DATABASE WPI Week 9802 Derwent Publications Ltd., London, GB; AN 98-011903 XP002089108 & JP 09 255518 A (NOEVIR KK), 30 septembre 1997 (1997-09-30)
- DATABASE WPI Week 8715 Derwent Publications Ltd., London, GB; AN 87-104725 XP002089109 & JP 62 051606 A (SHISEIDO CO. LTD.), 6 mars 1987 (1987-03-06)

## Description

La présente invention a pour objet une nouvelle composition cosmétique de composés à structure lipoaminoacide, à activité apaisante.

Dans la vie moderne, la peau est agressée par divers facteurs externes au corps humain; il s'agit notamment de la pollution atmosphérique, des rayonnements ultraviolets qu'ils soient naturels ou artificiels ou d'autres stress tels que les stress mécaniques ou chimiques.

Du fait de la plus en plus grande prise en compte de ces problèmes, notamment dans les lieux fortement urbanisés, l'aspect protection de la peau est devenu prépondérant dans la recherche de nouveaux produits cosmétiques. En réponse aux agressions ou aux sensations d'agression de la peau, on a développé le concept de produit cosmétique apaisant.

Selon la directive du conseil de la Communauté Economique Européenne N°76/768/CEE du 27 Juillet 1976 modifiée par la directive N°93/35/CEE du 14 Juin 1993, on entend par "produit cosmétique" toute substance ou préparation destinée à être mise en contact avec les diverses parties superficielles du corps humain (épiderme, système pileux et capillaire, ongles, lèvres et organes génitaux) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état.

De manière générale, on appelle produit apaisant ou formulation cosmétique apaisante, tout produit en formulation qui procure une sensation de bien-être de la peau que ce soit notamment une sensation de douceur, d'élasticité, et/ou de réconfort ressentie par le sujet grâce à l'application dudit produit sur sa peau.

La Demanderesse a découvert que tous ces stress de la peau induisent un état pré-inflammatoire.

Lors de cet état, divers événements vont survenir, notamment la production de radicaux libres et la synthèse de divers enzymes pro inflammatoires, telle que l'élastase. Ces deux phénomènes (production de radicaux libres et d'élastase), vont, non seulement entretenir et propager cet état d'inflammation, mais aussi avoir des conséquences néfastes au niveau de la peau. La production de radicaux libres, par exemple, sera responsable de la mort de nombreuses cellules, à cause des effets délétères induits notamment au niveau de molécules primordiales telles que l'ADN, les lipides, ou les protéines; l'élastase, quant à elle, va dégrader l'élastine, principale protéine du soutien dermique. Ces deux phénomènes vont donc traduire ce qui se passe lors d'une inflammation ainsi que les effets néfastes cutanés qui en découlent.

La Demanderesse a donc cherché à développer une composition efficace d'une part contre le stress cutané provoqué par les agressions externes de la peau, notamment contre les méfaits provoqués par les enzymes qui entretiennent et développent le processus inflammatoire, et d'autre part en inhibant le processus de productions de radicaux libres.

Les composés à structure lipoaminoacide, comme par exemple ceux décrits dans les demandes intemationales de brevet publiées sous les numéros WO92/20647, WO92/21318, WO94/26694 et WO94/27561, sont, en raison de leur structure amphiphile, des vecteurs biologiques particulièrement intéressants en tant que régulateurs de la physiologie cutanée et s'avèrent appropriés à de multiples applications, notamment en cosmétique.

Or, la Demanderesse a trouvé que les compositions comprenant comme principes actifs, l'association de lipoaminacides avec certains extraits naturels de plantes, possédaient à la fois une activité antiradicalaire et une activité anti-élastase. La demanderesse a aussi trouvé que cette activité était le résultat de la synergie entre ces deux familles de principes actifs.

L'invention a pour objet une composition caractérisée en ce qu'elle comprend à titre de principe actif, au moins un composé de formule (I): 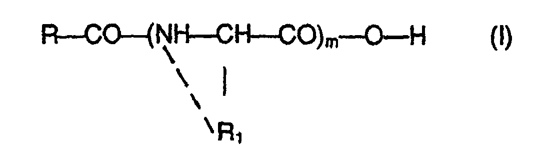 ou ses sels topiquement acceptables, dans laquelle R représente la chaîne caractérisante d'un acide gras, saturé ou insaturé, linéaire ou ramifié, comportant de 3 à 30 atomes de carbone, R₁ représente la chaîne caractérisante d'un acide aminé et m est compris entre 1 et 5,
et les constituants d'au moins un extrait et/ou d'au moins une teinture de plantes de la famille des nymphéacées.

Par sel topiquement acceptable, on entend tout sel de l'acide de formule (I) biologiquement acceptable pour la peau et/ou les muqueuses, c'est à dire tout sel pouvant notamment régler le pH de la composition à une valeur comprise entre 3 et 8 et de préférence environ égale à 5, c'est à dire à un pH voisin de celui de la peau II peut s'agir notamment de sels alcalins tels que les sels de sodium, de potassium ou de lithium, de sels alcalino-terreux tels que les sels de calcium, de magnésium ou de strontium; il peut aussi s'agir de sels métalliques tels que les sels divalents de zinc ou de manganèse ou encore les sels trivalents de fer, de lantane, de cérium ou d'aluminium.

Le composé de formule (I) présent dans la composition objet de la présente invention, peut être sous forme d'acide libre ou sous forme partiellement ou totalement salifiée.

L'expression "chaîne caractérisante" utilisée dans le cadre de la présente demande de brevet, désigne la chaîne principale non fonctionnelle de l'acide gras ou de l'acide aminé considéré.

Ainsi, pour un acide gras répondant à la formule générale R-COOH, la chaîne caractérisante sera la chaîne représentée par R. Le radical R représente notamment un radical comportant de 5 à 22 atomes de carbone choisi parmi les radicaux pentyle, hexyle, heptyle, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle. L'invention a plus particulièrement pour objet la composition telles que décrite précédemment pour laquelle, dans la formule (I), le fragment R-CO comporte de 7 à 22 atomes de carbone et représente notamment l'un des radicaux hexanoyle, heptanoyle, octanoyle (capryloyle), décanoyle (caproyle), undécylènoyte, dodécanoyle (lauroyle), tétradécanoyle (myristyle), hexadécanoyle (palmitoyle), octadécanoyle (stéaryle), eicosanoyle (arachidoyle), docosanoyle (behènoyle), octodécènoyle (oléyle), éicosènoyle (gadoloyle), docosènoyle (érucyle), octadécadiènoyle (linolènoyle). Dans une première variante préférée de la présente invention, dans la formule (I), le fragment R-CO (I) comporte de 14 à 18 atomes de carbone.

Pour un acide aminé représenté par la formule générale

H₂N-CHR₁-COOH,

comme pour un acide aminé représenté par la formule: 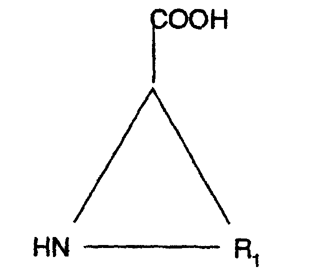 la chaîne caractérisante sera la chaîne représentée par R₁. R₁ représente notamment la chaîne caractérisante d'un des acides aminés choisis parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la cystéine, la cystine, la méthionine, l'hydroxyproline, l'hydroxylysine et l'omithine.

L'invention a plus particulièrement pour objet la composition telles que décrite précédemment pour laquelle, dans la formule (I), R₁ représente la chaîne caractérisante de la proline.

Par au moins un composé de formule (I), on indique que la composition selon l'invention peut contenir un ou plusieurs de ces composés.

Dans la définition de la composition objet de la présente invention, les mots extrait et teinture sont utilisés dans leurs sens respectifs tels qu'ils sont établis dans l'édition de 1997 de la Pharmacopée Européenne ; les extraits (extracts) sont des préparations concentrées, liquides, solides ou de consistance intermédiaire, généralement obtenues à partir de matières premières végétales ou animales séchées. Les teintures (tincturae) sont des préparations généralement obtenues à partir de matières premières végétales ou animales séchées.

Par extraits ou teintures de plantes de la famille des nymphéacées, on désigne notamment les extraits ou teintures de nénuphar tel que " Nuphar japonicum ", de lotus tel que " Nelumbo nucifera " ou de " Brasenia purpurea ". Ces extraits ou teintures sont disponibles dans le commerce. Certains sont inscrits aux pharmacopées française et/ou européenne. L'invention a plus particulièrement pour objet la composition telle que décrite précédemment dans laquelle l'extrait ou teinture de plantes de la famille des nymphéacés est un extrait de nénuphar et, plus particulièrement, un extrait de fleur de nénuphar.

Les composés de formule (I) sont généralement obtenus par acylation de composés de formule (I') 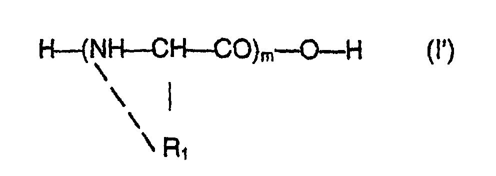 ou de leur sels, eux-mêmes obtenus par hydrolyse totale ou partielle de protéines de toutes origines. Ces protéines peuvent être d'origine animale, telles que, par exemple, le collagène, l'élastine, la protéine de chair de poissons, la gélatine de poissons, la kératine ou la caséine, d'origine végétale, comme les protéines de céréales, de fleurs ou de fruits, telles que par exemple, les protéines issues du soja, du tournesol, de l'avoine, du blé, du maïs, de l'orge, de la pomme de terre, du lupin, de la féverolle, de l'amande douce, du kiwi, de la mangue ou de la pomme; il peut s'agir aussi de protéines obtenues à partir de chorelles (algues unicellulaires), d'algues roses, de levures ou de la soie. Cette hydrolyse peut être réalisée par exemple par chauffage à des températures comprises entre 60 et 130°C d'une protéine placée dans un milieu acide ou alcalin. Cette hydrolyse peut également être réalisée par voie enzymatique avec une protéase, couplée éventuellement à une post-hydrolyse alcaline ou acide.

Quand m est supérieur à 1, R₁ représente plusieurs des chaînes caractérisantes des acides aminés, selon la protéine hydrolysée et le degré d'hydrolyse.

Dans une variante préférée de la présente invention, lorsque la composition ne comprend qu'un seul composé de formule (I), m est égal à 1 et lorsque la composition comprend un mélange de composés de formule (I), le degré moyen de condensation des acides aminés N-acylée dans ce mélange est inférieur à 2.

La réaction d'acylation permettant d'obtenir les composés de formule (I) précitée peut être réalisée par voie chimique en milieu alcalin (pH de 8 à 10) selon la réaction de Schotten Bauman ou par voie enzymatique et l'homme de métier pourra se reporter notamment à la référence Surfactant Science Series, volume 7, Anionic Surfactants, partie II, chapitre 16, pages 581 à 617 (Marcel Dekker - 1976). D'une façon générale, le mode de réalisation actuellement préféré pour la préparation des composés lipoaminoacides de formule (I) comprend les étapes suivantes :
a) Acylation en milieu alcalin (pH 8 à 10) d'un excès de mélange d'acides aminés (mélange extemporané ou obtenu par hydrolyse complète d'une protéine) par un acide gras (ou un mélange d'acides gras), sous forme de chlorure d'acide ou d'anhydride. Le rapport acides aminés/chlorure acide est de préférence de 1,05 à 1,30 équivalents. La température d'acylation optimale se situe vers 80° C mais varie d'un acide aminé à l'autre entre 60 et 110° C. La durée d'acylation dépend de l'équipement utilisé (taille, agitation); elle est de 2 heures environ pour une masse acylée de 500 kg et de 5 heures environ pour une masse acylée de 5 000 kg.
b) Cassage de l'acylat alcalin par acidification pour décanter les impuretés solubles dans l'eau et relarguer l'acylat organique acide (pH optimal de 0,5 à 3 selon les acides aminés).
c) Purification par lavage à l'eau ou avec addition d'électrolytes ou de co-solvant pour favoriser la décantation.

Outre les principes actifs, la composition selon l'invention comprend des véhicules minéraux ou organiques couramment utilisés dans la fabrication de compositions destinées à être formulées en préparations à usage cosmétique et/ou pharmaceutique; on peut citer par exemple l'eau, les polyols comme le propylèneglycol, le dipropylène glycol, le butylèneglycol, l'hexylèneglycol ou la glycérine.

Dans un aspect préféré de la présente invention la composition telle que décrite précédemment comprend de 15% à 60% et, plus particulièrement, de 20% à 40% en poids d'au moins un composé de formule (I) dans laquelle le fragment R-CO comprend de 8 à 18 atomes de carbone, ou ses sels topiquement acceptables, et de 0,01% à 10% en poids et, plus particulièrement, de 0,01% à 5% en poids de constituants d'au moins un extrait et/ou une teinture de plantes de la famille des nymphéacées.

Selon la Pharmacopée Européenne, les extraits peuvent être sous forme d'extraits fluides, d'extraits mous ou fermes ou d'extraits secs.
Dans la définition précédente de la composition selon l'invention, les pourcentages pondéraux en constituants de l'extrait ou de la teinture correspondent aux pourcentages pondéraux en résidu sec, ledit résidu sec étant obtenu par évaporation du solvant et dessiccation dudit extrait ou de ladite teinture à des conditions opératoires auxquelles l'altération des constituants est minime.

La composition telle que définie précédemment comprend notamment de 20% à 40% en poids de N-palmitoyl proline et de 0,01% à 1% en poids de constituants d'un extrait de nénuphar et, plus particulièrement, de fleur de nénuphar. La composition selon l'invention peut aussi comprendre de 0,1% à 10% en poids d'aspartate de magnésium et de potassium.

Dans une autre variante de la présente invention, la composition telle que décrite précédemment comprend en outre de 0,1% à 10% en poids de gluconate de zinc.

La composition, objet de la présente invention est préparée par des méthodes connues de l'homme du métier. En général, on introduit sous agitation, dans une composition comprenant au moins un composé de formule (I), au moins un extrait et/ou une teinture de plantes, en quantité appropriée pour atteindre la concentration souhaitée dans la solution finale en constituants dudit extrait et/ou de ladite teinture. Si nécessaire, ou si désiré, ledit extrait et/ou la dite teinture sont préalablement dilués avant mélange.

La composition selon l'invention est utilisée en cosmétique. Comme le montrent les exemples suivants, la composition selon l'invention se caractérise de façon inattendue par une double activité, anti-élastase et anti radicalaire. Ceci permet de l'utiliser notamment, pour apaiser et/ou protéger les peaux sensibles, ou pour apaiser les peaux soumises à l'exposition au soleil, hydrater les peaux sèches, ralentir le vieillissement de la peau et /ou traiter les peaux à tendance acnéique; dans cette dernière indication la composition selon l'invention peut être utilisée comme traitement complémentaire au traitement médical de l'acné.

La composition selon l'invention est aussi utilisée lors d'un simple acte d'hygiène corporelle ou d'un traitement complémentaire au traitement médical d'une infection.

La composition selon l'invention est aussi utilisée dans le traitement du cuir chevelu notamment comme actif antipelliculaire.

Ces utilisations constituent aussi en elles-mêmes un objet de la présente invention.

Selon l'utilisation, la composition telle que décrite précédemment est mise en oeuvre à des concentrations différentes et dans une formulation appropriée à cette utilisation; de telles compositions cosmétiques se présentent habituellement sous forme de solutions aqueuses, de solutions alcooliques diluées, ou d'émulsions simples ou multiples, telles que les émulsion eau dans huile (E/H), huile dans eau (H/E) ou eau dans huile dans eau (E/H/E) dans lesquelles les huiles sont de nature végétale ou minérale, comme huile de nature minérale, on peut citer les silicones. Comme formulation cosmétique on peut citer, les crèmes, les laits, les lotions toniques ou démaquillantes, les lingettes, les gels douches, les fonds de teint, les savons, les savons liquides, les syndets, les produits d'hygiène intime, les shampooings, les produits déodorants ou les produits de maquillage.

De telles formulations sont connues de l'homme du métier; leur préparation sont décrites, par exemple, dans les demandes de brevet publiées sous les numéros, WO92/0677 WO93/28204, WO95/13863, WO95/35089, ou WO96/22109.

L'invention a donc aussi pour objet une formulation cosmétique susceptible d'être obtenue par dilution du 1/10 jusqu'au 1/20000, de préférence du 1/10 au 1/100, de la composition telle que décrite précédemment, dans un ou plusieurs excipients cosmétiquement acceptables, et notamment une formulation cosmétique sous forme d'une émulsion huile dans eau ayant l'aspect d'un lait ayant une viscosité inférieure à 1Pa.s. comprenant comme émulsionnant une composition auto-émulsionnable à base d'alcools gras.

Comme compositions auto-émulsionnables préférées, on peut citer les MONTANOV® 68, MONTANOV® 14, MONTANOV® 82 et MONTANOV® 202 commercialisés par la société SEPPIC.

Selon le caractère que l'on peut donner à la formulation cosmétique, on peut le cas échéant ajouter un latex inverse tels que le SEPIGEL®305, le SEPIGEL® 501 ou le SIMULGEL® 600. Le terme dilution employé dans ce qui précède, englobe dans son acception la plus large, toutes les étapes permettant de passer de la composition telle que définie précédemment à la formulation cosmétique destinée à être commercialisée. Dans un autre mode préféré de la présente invention, la formulation cosmétique est une crème ou un lait apaisant pour traiter les peaux sensibles et particulièrement la peau du visage. Dans un autre mode préféré de la présente invention, la formulation cosmétique est une formule moussante ou un shampooing.

L'invention a particulièrement pour objet une formulation cosmétique comprenant à titre de principe actif de 0,001% à 6 % en poids d'au moins un composé de formule (I) et de 0,00005% à 1% en poids de constituants d'au moins un extrait (II) et/ou d'au moins une teinture de plante de la famille des nymphéacées et si désiré jusqu'à 1% de gluconate de zinc.

L'invention a tout particulièrement pour objet une formulation cosmétique comprenant à titre de principe actif de 0,1% à 2 % en poids de palmitoyl proline et de 0,0001% à 0,01% en poids de constituants d'au moins un extrait de nénuphar et, plus particulièrement, de fleur de nénuphar.

Selon les parties superficielles du corps humain auxquelles s'applique le traitement cosmétique mis en oeuvre avec la formulation telle que définie précédemment, et le selon le traitement cosmétique qui est promu pour ladite formulation, celle-ci pourra contenir, outre la composition synergique objet de la présente invention, d'autre principes actifs usuellement mis en oeuvre en cosmétique.

La formulation telle que définie précédemment, peut ainsi contenir en outre, un ou plusieurs lipoaminoacides en complément que ceux déjà initialement contenus dans la composition objet de la présente invention.
Dans aspect particulier de la formulation précédente, celle-ci comprend en outre de 0,1% à 1% en poids d'undécylènoyl glycine et/ou de 0,1% à 1% en poids d'octanoyl glycine.
Les exemples suivants illustrent l'invention sans toutefois la limiter.

### Exemple 1:

### A) Préparation d'une composition selon l'invention

On a préparé une composition A comprenant comme principe actif environ 30% de palmitoyl proline sous forme de sel de sodium et 0,1% d'extrait de fleur de nénuphar, à partir de palmitoyl proline et d'un extrait glycolique (butylène glycol) de fleur de nénuphar (nymphea alba) commercialisé par la Société ALBAN MULLER INTERNATIONAL; le pH le de la composition A est d'environ 5,8.

### B) Mise en évidence des propriétés anti-élastase de la composition selon l'invention

### a) Principe du test :

On sait que l'élastase leucocytaire humaine (ELH) intervient dans un grand nombre de pathologies inflammatoires. Cet enzyme est en particulier capable de dégrader de nombreuses macromolécules telles que l'élastine fibreuse, certains types de collagène, les protéoglycanes, les glycoprotéines. Pour cette raison, l'élastase leucocytaire humaine constitue l'un des maillons de la chaîne des réactions accompagnant un phénomène d'inflammation. Le blocage de cet enzyme par un effet anti-élastase permet donc d'empêcher la dégradation des molécules précitées et donc d'inhiber le processus de l'inflammation. Les propriétés anti-élastasiques d'un produit donné, peuvent être mises en évidence par un test in-vitro, réalisé avec un spectrophotomètre, en utilisant une substance support susceptible de se dégrader en se colorant, au contact de l'élastase leucocytaire humaine.
Une telle substance peut être par exemple la N-méthoxysuccinyl-alanine-proline-valine-para-nitroanilide, substance normalement incolore qui libère, par hydrolyse par l'élastase leucocytaire humaine, un produit coloré, la paranitroalinine, dont la cinétique d'apparition peut être suivie par spectrophotométrie à 410 nm. La réaction est réalisée dans un spectrophotomètre thermostaté à 25°C, disposant d'un passeur d'échantillons. Toutes les cinétiques sont réalisées au minimum trois fois, la moyenne et l'écart-type étant alors calculés pour les trois valeurs obtenues. La présence d'une molécule à activité anti-élastasique se traduit par une limitation de l'apparition du produit coloré et l'effet anti-élastasique peut être alors calculé par rapport à une courbe témoin obtenue en l'absence de ladite molécule. Il existe ainsi une corrélation entre le pourcentage d'inhibition de l'apparition du produit coloré par le composé testé et le pourcentage d'inhibition de l'élastase leucocytaire humaine. Le pourcentage d'inhibition ainsi calculé est également représentatif de l'activité apaisante du composé testé.

### b) Résultats obtenus

La composition A est diluée dans l'eau pour obtenir la composition A₁, ayant les concentrations en principe actives suivantes :

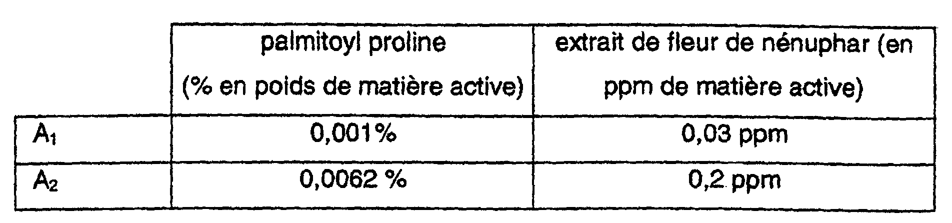

Les pourcentages d'inhibition de l'élastase sont les suivants :

| Produits | Concentrations testées: | % d'inhibition anti-élastase |
|---|---|---|
| palmitoyl proline seul | 0.001 % | 22 % |
| extrait de fleur de nénuphar seul | 0.03 ppm | 28% |
| palmitoyl proline + extrait de fleur de nénuphar | 0.001 % de palmitoyl proline + 0.03 ppm d'extrait de fleur de nénuphar | 63% (effet additif=50%) ⇒ Synergie présente |

Ces résultats montrent qu'à des concentrations pour lesquelles le palmitoyl proline et l'extrait de fleur de nénuphar ont une activité limitée (de 22% et 28 %), la combinaison des deux présente de façon inattendue une activité supérieure (63% d'inhibition).

### C) Mise en évidence des propriétés anti-radicalaires de la composition selon l'invention.

### a) Principe du test

La détermination de l'effet antiradicalaire est basée sur l'inhibition ou la diminution de la vitesse de réduction du cytochrome C, par l'ajout au milieu réactionnel d'une molécule à étudier. L'anion superoxyde est engendré par l'action de la xanthine oxydase sur la xanthine. Il entraîne, en l'absence d'une molécule capable de le capter, la réduction du cytochrome C. L'apparition de cytochrome C réduit est suivie au spectrophotomètre, à 550 nm en présence (Essai) et en absence (Témoin) de molécules antiradicalaires.

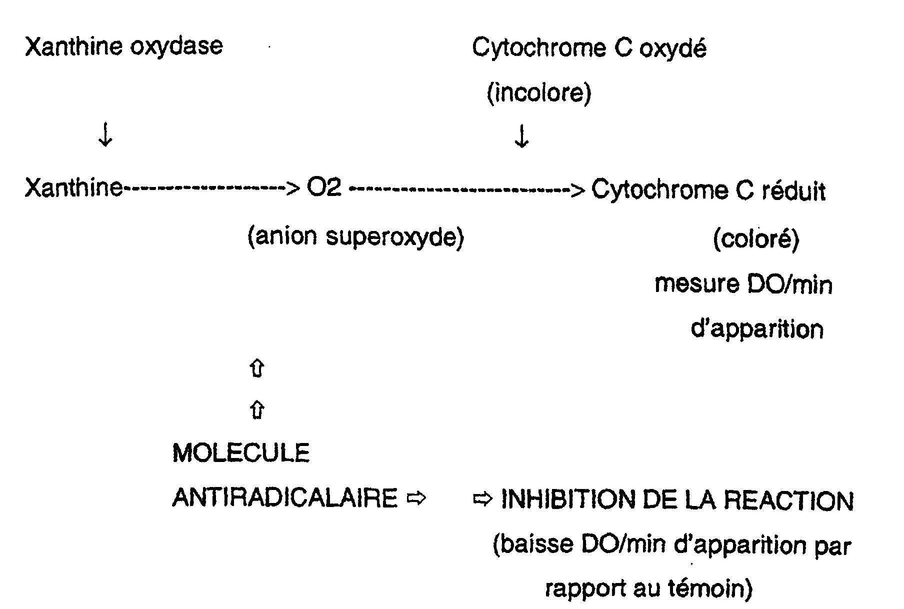

La réaction se fait dans un spectrophotomètre thermostaté à 25°C et disposant d'un passeur d'échantillons. Toutes les cinétiques sont réalisées au minimum trois fois ; la moyenne et l'écart type sont calculés pour les trois valeurs obtenues. Un pourcentage d'inhibition de la vitesse d'apparition du produit coloré (correspondant à la quantité d'anion superoxyde libre) sera donc calculé pour chaque actif testé. Ce calcul sera effectué par rapport à la vitesse d'apparition du produit coloré dans le témoin (sans actif). Le pourcentage d'inhibition de l'apparition du produit coloré par l'actif correspondra donc à son pourcentage d'inhibition de l'anion superoxyde.

### b) Résultats obtenus

| Produits | Concentrations testées | % d'inhibition antiradicalaire |
|---|---|---|
| palmitoyl proline seul | 0.0062% | 38% |
| extrait de fleur de nénuphar seul | 0,2 ppm | 20% |
| palmitoyl proline + extrait de fleur de nénuphar | 0.0062 %palmitoyl proline + 0,2 ppm d'extrait de fleur de nénuphar | 74% (effet additif=58%) ⇒ Synergie présente. |

Ces résultats font apparaître une synergie de l'activité antiradicalaire inhérente à la combinaison de deux produits.

Ces essais montrent l'avantage qu'il y a, à combiner dans une même formulation, un produit de formule (I) et un extrait de plantes de la famille des nymphéacées.

### Exemples de formulation cosmétiques (Exemples 2 à 32)

### Exemple 2: Crème de soin

| | |
|---|---|
| Cyclométhicone | 10% |
| Composition A | 3% |
| SEPIGEL® 501 | 0,8% |
| MONTANOV®68 | 2% |
| alcool stéarylique | 1% |
| alcool stéarique | 0,5% |
| conservateur | 0,65% |
| Lysine | 0,025% |
| EDTA (sel disodique) | 0,05% |
| Gomme de xanthane | 0,2% |
| Glycérine | 3% |
| eau | q.s.p. 100% |
| (Le MONTANOV® 68 (cétéaryl glucoside), est une composition auto-émulsionnable telle que décrite dans WO 92/06778, commercialisée par la société SEPPIC) (Le SEPIGEL® 501 est un agent épaississant à base de copolymères d'acrylamide commercialisé par la société SEPPIC) | |

### Exemple 3: Crème de soin

| | |
|---|---|
| Cyclométhicone | 10% |
| Composition A | 3% |
| SEPIGEL® 501 | 0,8% |
| MONTANOV® 68 | 2% |
| Perfluoropolymethylisopropylether | 0,5% |
| alcool stéarylique | 1% |
| alcool stéarique | 0,5% |
| conservateur | 0,65% |
| Lysine | 0,025% |
| EDTA (sel disodique) | 0,05% |
| PEMULEN®TR | 0,2% |
| Glycérine | 3% |
| eau | q.s.p. 100% |
| (Le PEMULEN®TR est un polymère acrylique commercialisé par GOODRICH). | |

### Exemple 4: Baume après-rasage

| FORMULE | | |
|---|---|---|
| A | Composition A | 3% |
| | SEPIGEL® 501 | 1,5% |
| | eau | q.s.p 100% |
| | | |
| B | MICROPEARL® M 100 | 5,0% |
| | SEPICIDE® CI | 0,50% |
| | Parfum | 0,20% |
| | éthanol 95° | 10,0% |
| (Le MICROPEARL® M 100 est une poudre ultra fine au toucher très doux et à action matifiante commercialisée par la société MATSUMO) (Le SEPICIDE® Cl ,imidazolin urée, est un agent conservateur commercialisé par la société SEPPIC) | | |

### MODE OPERATOIRE

### Ajouter B dans A.

### Exemple 5: Emulsion satinée pour le corps

| FORMULE | | |
|---|---|---|
| A | SIMULSOL® 165 | 5,0% |
| | LANOL® 1688 | 8,50% |
| | beurre de Karité | 2% |
| | huile de parafine | 6,5% |
| | LANOL® 14M | 3% |
| | LANOL® S | 0,6% |
| | | |
| B | eau | 66,2% |
| | | |
| C | MICROPEARL® M 100 | 5% |
| | | |
| D | Composition A | 3% |
| | SEPIGEL® 501 | 3% |
| | | |
| E | SEPICIDE® CI | 0,3% |
| | SEPICIDE® HB | 0,5% |
| | MONTEINE® CA | 1% |
| | parfum | 0,20% |
| | acétate de vitamine E | 0,20% |
| | Sodium pyrolidinone carboxylate | 1% (agent hydratant) |
| (Le SIMULSOL® 165 (stéarate de glycérol/stéarate de PEG100) est une composition auto-émulsionnable commercialisée par la société SEPPIC) (Le LANOL® 1688 est un ester émollient à effet non gras commercialisé par la société SEPPIC) (Le LANOL® 14M et le LANOL® S sont des facteurs de consistance commercialisés par la société SEPPIC) (Le SEPICIDE® HB ,(mélange de phénoxyéthanol/méthyl paraben/éthylparaben/ propylparaben/butylparaben), est un agent conservateur commercialisé par la société SEPPIC) (Le MONTEINE® CA est un agent hydratant commercialisé par la société SEPPIC) | | |

### MODE OPERATOIRE

### Ajouter C dans B, émulsionner B dans A à 70°C, puis ajouter D à 60°C puis E à 30°C.

### Exemple 6: Lait corporel

| FORMULE | | |
|---|---|---|
| A | SIMULSOL® 165 | 5,0% |
| | LANOL® 1688 | 12,0% |
| | LANOL® 14M | 2,0% |
| | alcool cétylique | 0,3% |
| | SCHERCEMOL® OP | 3% |
| | | |
| B | eau | q.s.p. 100% |
| | | |
| C | Composition A | 3% |
| | SEPIGEL® 501 | 0,35% |
| | | |
| D | SEPICIDE® CI | 0,2% |
| | SEPICIDE® HB | 0,5% |
| | parfum | 0,20% |
| (Le SCHERCEMOL® OP est un ester émollient à effet non gras) | | |

### MODE OPERATOIRE

### Emulsionner B dans A vers 75°C ; ajouter C vers 60°C, puis D vers 30°C

### Exemple 7: crème H/E

| FORMULE | | |
|---|---|---|
| A | SIMULSOL®165 | 5,0% |
| | LANOL®1688 | 20,0% |
| | LANOL® P | 1,0% (additif à effet stabilisant) |
| | | |
| B | eau | q.s.p. 100% |
| | | |
| C | Composition A | 3% |
| | SEPIGEL® 501 | 2,5% |
| | | |
| D | SEPICIDE® CI | 0,20% |
| | SEPICIDE® HB | 0,30% |
| (Le LANOL® P est un additif à effet stabilisant commercialisé par la société SEPPIC) | | |

### MODE OPERATOIRE

### Introduire B dans A vers 75°C ; ajouter C vers 60°C, puis D vers 45°C

### Exemple 8: gel solaire non gras

| FORMULE | | |
|---|---|---|
| A | Composition A | 3% |
| | SEPIGEL® 501 | 0,8% |
| | eau | 30% |
| | | |
| B | SEPICIDE® Cl | 0,20% |
| | SEPICIDE® HB | 0,30% |
| | parfum | 0,10% |
| | | |
| C | colorant | q.s. |
| | eau | 30% |
| | | |
| D | MICROPEARL® M 100 | 3,00% |
| | eau | q.s.p 100% |
| | | |
| E | huile de silicone | 2,0% |
| | PARSOL® MCX | 5,00% |
| (Le PARSOL® MCX est de l'octyl paraméthoxycinnamate; il est commercialisé par la société GIVAUDAN) | | |

### MODE OPERATOIRE

### Introduire B dans A; ajouter C, puis D, puis E.

### Exemple 9: Lait solaire

| FORMULE | | |
|---|---|---|
| A | SEPIPERL®N | 3,0% |
| | huile de sésame | 5,0% |
| | PARSOL® MCX | 5,0% |
| | Carraghénane λ | 0,10% |
| | | |
| B | eau | q.s.p.100% |
| | | |
| C | Composition A | 3% |
| | SEPIGEL® 501 | 0,8% |
| | | |
| D | parfum | q.s. |
| | conservateur | q.s. |
| (Le SEPIPERL®N est un agent nacrant, commercialisé par la société SEPPIC, à base d'un mélange d'alkyl polyglucosides tels que ceux décrits dans WO 95/13863) | | |

### MODE OPERATOIRE

### Emulsionner B dans A à 75°C puis ajouter C vers 60°C, puis D vers 30°C et ajuster le pH si nécessaire

### Exemple 10: Gel de massage

| FORMULE | | |
|---|---|---|
| A | Composition A | 3% |
| | SEPIGEL® 501 | 3,5% |
| | eau | 20,0% |
| | | |
| B | colorant | 2 gouttes/100g |
| | eau | q.s. |
| | | |
| C | alcool | 10% |
| | menthol | 0,10% |
| | | |
| D | huile de silicone | 5,0% |

### MODE OPERATOIRE

### Ajouter B dans A; puis ajouter au mélange, C puis D

### Exemple 11: gel soin de massage

| FORMULE | | |
|---|---|---|
| A | Composition A | 3% |
| | SEPIGEL® 501 | 3,0% |
| | eau | 30% |
| | | |
| B | SEPICIDE® Cl | 0,20% |
| | SEPICIDE® HB | 0,30% |
| | parfum | 0,05% |
| | | |
| C | colorant | q.s. |
| | eau | q.s.p 100% |
| | | |
| D | MICROPEARL® SQL | 5,00% |
| | LANOL® 1688 | 2% |
| (Le MICROPEARL® SQL est un mélange de microparticules renfermant du squalane qui se libère sous l'action du massage; il est commercialisé par la société MATSUMO) | | |

### MODE OPERATOIRE

### Préparer A; additionner B, puis C, puis D.

### Exemple 12: Gel coup d'éclat

| FORMULE | | |
|---|---|---|
| A | Composition A: | 3% |
| | SEPIGEL® 501: | 4% |
| | eau: | 30% |
| | | |
| B | ELASTINE HPM: | 5,0% |
| | | |
| C | MICROPEARL® M 100: | 3% |
| | eau: | 5% |
| | | |
| D | SEPICIDE® CI: | 0,2% |
| | SEPICIDE® HB: | 0,3% |
| | Parfum: | 0,06% |
| | Sodium pyrolidinone carboxylate | 50%: 1% |
| | eau: | q.s.p. 100% |

### MODE OPERATOIRE

### Préparer A; additionner B, puis C, puis D.

### Exemple 13: Lait corporel

| FORMULE | | |
|---|---|---|
| A | SEPIPERL®N | 3,0% |
| | Triheptonate de glycerol | 10,0% |
| | | |
| B | eau | q.s.p.100% |
| | | |
| C | Composition A: | 1,5% |
| | SEPIGEL® 501: | 1,0% |
| | | |
| D | parfum: | q.s. |
| | conservateur: | q.s. |

### MODE OPERATOIRE

### Fondre A à environ 75°C. Emulsionner B dans A à 75°C puis ajouter C vers 60°C, puis D.

### Exemple 14: Emulsion démaquillante à l'huile d'amande douce

| FORMULE | |
|---|---|
| MONTANOV® 68 | 5% |
| huile d'amandes douces | 5% |
| eau | q.s.p.100% |
| Composition A | 1% |
| SEPIGEL® 501 | 0,3% |
| glycérine | 5% |
| conservateur | 0,2% |
| parfum | 03% |

### Exemple 15: Crème hydratante pour peaux grasses

| FORMULE | |
|---|---|
| MONTANOV® 68 | 5% |
| cétylstéaryloctanoate | 8% |
| octyl palmitate | 2% |
| eau | q.s.p.100% |
| Composition A | 2% |
| SEPIGEL® 501 | 0,6% |
| MICROPEARL® M100 | 3,0% |
| Mucopolysaccharides | 5% |
| SEPICIDE® HB | 0,8 |
| parfum | 03% |

### Exempte 16: Baume après-rasage apaisant sans alcool

| FORMULE | |
|---|---|
| mélange de lauryl aminoacides | 0,1% à 5% |
| aspartate de magnésium et de potassium | 0,002% à 0,5% |
| LANOL® 99 | 2% |
| huile d'amandes douces | 0,5% |
| eau | q.s.p.100% |
| Composition A | 3% |
| SEPIGEL® 501 | 3% |
| SEPICIDE® HB | 0,3% |
| SEPICIDE® Cl | 0,2% |
| parfum | 0,4% |
| (Le LANOL® 99 est de l'isononyl isononanoate commercialisé par la société SEPPIC). | |

### Exemple 17: Crème aux AHA pour peaux sensibles

| FORMULE | |
|---|---|
| LANOL® 99 | 2% |
| MONTANOV® 68 | 5,0% |
| eau | q.s.p. 100% |
| Composition A | 0,1 à 5% |
| SEPIGEL® 501 | 1,5% |
| acide gluconique | 1,50% |
| triéthylamine | 0,9% |
| SEPICIDE® HB | 0,3% |
| SEPICIDE® Cl | 0,2% |
| parfum | 0,4% |

### Exemple 18: Soin apaisant après soleil

| FORMULE | |
|---|---|
| LANOL® 99 | 10,0% |
| eau | q.s.p.100% |
| Composition A | 3% |
| SEPIGEL® 501 | 2,5% |
| SEPICIDE® HB | 0,3% |
| SEPICIDE® Cl | 0,2% |
| parfum | 0,4% |
| colorant | 0,03% |

### Exemple 19: Lait démaquillant

| FORMULE | |
|---|---|
| SEPIPERL®N | 3% |
| PRIMOL 352: | 8,0% |
| huile d'amandes douces: | 2% |
| eau: | q.s.p. 100% |
| Composition A: | 2% |
| SEPIGEL® 501: | 0,8% |
| conservateur: | 0,2% |

### Exemple 20: Lait corporel

| FORMULE | |
|---|---|
| SEPIPERL®N | 3,5% |
| LANOL® 37T | 8,0% |
| SOLAGUM®L | 0,05% |
| eau | q.s.p.100% |
| benzophénone | 2,0% |
| diméthicone 350cPs | 0,05% |
| Composition A | 2% |
| SEPIGEL® 501 | 0,8% |
| conservateur | 0,2% |
| parfum | 0,4% |
| (Le LANOL® 37T est du triheptanoate de glycérol, commercialisé par la société SEPPIC). (Le SOLAGUM®L, est un carraghénane commercialisé par la société SEPPIC). | |

### Exemple 21: émulsion fluide à pH alcalin

| | |
|---|---|
| MARCOL® 82 | 5,0% |
| NaOH | 10,0% |
| eau | q.s.p.100% |
| Composition A | 4% |
| SEPIGEL® 501 | 1,5% |
| (Le MARCOL® 82 est une huile paraffine commercialisée par la société ESSO) | |

### Exemple 22: Fond de teint fluide

| FORMULE | |
|---|---|
| SIMULSOL® 165 | 5,0% |
| LANOL® 84D: | 8,0% |
| LANOL® 99: | 5,0% |
| eau: | q.s.p.100% |
| pigments et charges minérales: | 10,0% |
| Composition A: | 3% |
| SEPIGEL® 501: | 1,2% |
| conservateur: | 0,2% |
| parfum: | 0,4% |
| (Le LANOL® 84D est du dioctylmalate commercialisé par la société SEPPIC). | |

### Exemple 23: Lait solaire

| FORMULE | |
|---|---|
| SEPIPERL®N | 3,5% |
| LANOL® 37T: | 10,0% |
| PARSOL NOX®: | 5,0% |
| EUSOLEX® 4360: | 2,0% |
| eau: | q.s.p.100% |
| Composition A: | 3% |
| SEPIGEL® 501: | 1,8% |
| conservateur: | 0,2% |
| parfum: | 0,4% |
| (Le PARSOL NOX® et I' EUSOLEX® 4360 sont deux filtres solaires commercialisés respectivement par les sociétés GIVAUDAN et MERCK). | |

### Exemple 24: Gel contour des yeux

| FORMULE | |
|---|---|
| Composition A: | 1% |
| SEPIGEL® 501: | 2% |
| Parfum: | 0,06% |
| Sodium pyrrolidinonecarboxylate: | 0,2% |
| DOW CORNING® 245 FLuid | 2,0% |
| eau: | q.s.p. 100% |
| (Le DOW CORNING® 245 FLuid est de la cyclométhicone, commercialisée par la société DOW CORNING): | |

### Exemple 25: composition de soin non rincée

| FORMULE | |
|---|---|
| Composition A | 3% |
| SEPIGEL® 501 | 1,5% |
| Parfum | q.s |
| conservateur | q.s. |
| DOW CORNING® X2 8360 | 5,0% |
| DOW CORNING® Q2 1401 | 15,% |
| eau | q.s.p. 100% |
| (Le DOW CORNING® 245 FLuid est de la cyclométhicone, commercialisée par la société DOW CORNING): | |

### Exemple 26: gel amincissant

| | |
|---|---|
| Composition A: | 5% |
| SEPIGEL® 501: | 5% |
| Ethanol | 30 % |
| Menthol | 0,1 % |
| Caféine | 2,5 % |
| extrait de ruscus | 2 % |
| extrait de lierre | 2 % |
| SEPICIDE®HP | 1 % |
| eau | q.s.p. 100 % |

### Exemple 27: Crème confort pour peaux sensibles

| | |
|---|---|
| Composition A: | 3% |
| SEPIGEL® 305: | 1% |
| MONTANOV® 68 | 7% |
| Isostéaryl isostéarate | 5% |
| Diméthicone | 10% |
| Parfum | 2% |
| SEPICIDE®HB | 0,3% |
| SEPICIDE™ CI | 0,2% |
| eau | q.s.p. 100 % |

### Exemple 28: Soin apaisant

| | |
|---|---|
| Composition A: | 3% |
| SEPIGEL® 305: | 1,5% |
| SIMULSOL® 165 | 5% |
| capric caprylic triglyceride | 5% |
| SEPICIDE®HB | 0,3% |
| SEPICIDE® CI | 0,2% |
| Parfum | 0,1% |
| eau | q.s.p. 100 % |

### Exemple 29: Gel crème pour peaux sensibles

| | |
|---|---|
| Composition A: | 3% |
| SEPIGEL® 305 | 2% |
| isohexadécane | 5% |
| huile de bourrache | 1% |
| SEPICIDE®HB | 0,3% |
| SEPICIDE® CI | 0,1% |
| eau | q.s.p. 100 % |

### Exemple 30: soin apaisant après soleil

| | |
|---|---|
| Composition A: | 3% |
| SEPIGEL® 501: | 4% |
| cyclométhicone et diméthiconol: | 5% |
| LANOL® 189: | 5% |
| SEPICIDE®HB | 0,3% |
| SEPICIDE® Cl | 0,2% |
| parfum | 0,2% |
| eau | q.s.p. 100 % |
| (Le LANOL® 189 est un ester de toucher riche et soyeux, commercialisé par la société SEPPIC). | |

### Exemple 31: Gel apaisant pour les mains

| | |
|---|---|
| Composition A: | 3% |
| SEPIGEL® 305: | 4% |
| Isostéaryl isostéarate: | 5% |
| MICROPEARL® M305: | 1% |
| glycérine: | 10% |
| SEPICIDE®HB | 0,3% |
| SEPICIDE® Cl | 0,2% |
| parfum | 0,2% |
| eau | q.s.p. 100 % |
| (Le MICROPEARL®M305 est un copolymère réticulé de polyméthyl méthacrylate est commercialisé par la société SEPPIC). | |

### Exemple 32: Crème hydratante pour peaux sensibles

| | |
|---|---|
| MONTANOV® 202: | 3% |
| Phytosqualane | 5% |
| Cyclométhicone: | 3% |
| Huile de Macadamia/Kiwi | 3% |
| eau: | q.s.p.100% |
| | |
| Composition A: | 3% |
| LIPACIDE ™ CSG | 0,5% |
| SEPIGEL® 305: | 1% |
| SEPICIDE® HB: | 0,2% |
| Parfum: | 0,05% |
| (Le MONTANOV® 202 (arachidyl glucoside/alcool behenylique) est une composition auto-émulsionnable telle que décrite dans WO 92/06778, commercialisée par la société SEPPIC) (Le LIPACIDE®C8G (octanoyl glycine) est commercialisé par la société SEPPIC) | |

### Exemple 33: soin auto-bronzant

| | |
|---|---|
| Composition A : | 3% |
| DHA | 1% |
| SEPIGEL® 305 : | 2% |
| MONTANOV® 202 | 3% |
| Huile d'amande douce | 7% |
| Diméthicone : | 3% |
| Parfum | 0,1% |
| SEPIFILM® HB | 0,3% |
| SEPIFILM® CI | 0,2% |
| eau: | q.s.p.100% |

## Revendications

1. Composition **caractérisée en ce qu'**elle comprend à titre de principe actif, au moins un composé de formule (I): 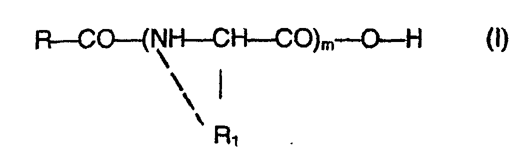 ou ses sels topiquement acceptables,
dans laquelle R représente la chaîne caractérisante d'un acide gras, saturé ou insaturé, linéaire ou ramifié, comportant de 3 à 30 atomes de carbone, R₁ représente une chaîne caractérisante d'un acide aminé et m est compris entre 1 et 5, et les constituants d'au moins un extrait et/ou d'au moins une teinture de plantes de la famille des nymphéacées.

2. Composition telle que définie à la revendication 1, pour laquelle, dans la formule (I), le fragment R-CO comporte de 7 à 22 atomes de carbone et représente notamment l'un des radicaux hexanoyle, heptanoyle, octanoyle (capryloyle), décanoyle (caproyle), undécylènoyle, dodécanoyle (lauroyle), tétradécanoyle (myristyle), hexadécanoyle (palmitoyle), octadécanoyle (stéaryle), eicosanoyle (arachidoyle), docosanoyle (behènoyle), octodécènoyle (oléyle), éicosènoyle (gadoloyle), docosènoyle (érucyle), octadécadiènoyle (linolènoyle).

3. Composition telle que définie à l'une des revendications 1 ou 2, pour laquelle dans la formule (I), le fragment R-CO (I) comporte de 14 à 18 atomes de carbone.

4. Composition telle que définie à l'une des revendications 1, 2 ou 3, pour laquelle dans la formule (I), R₁ représente la chaîne caractérisante de la proline.

5. Composition telle que définie à l'une des revendications 1 à 4, dans laquelle l'extrait ou teinture de plantes de la famille des nymphéacées est un extrait de nénuphar et plus particulièrement de fleur de nénuphar.

6. Composition telle que définie à l'une des revendications 1 à 5, qui comprend de 15 % à 60% et, plus particulièrement, de 20 % à 40 % en poids d'au moins un composé de formule (I) dans laquelle le fragment R-CO comprend de 8 à 18 atomes de carbone ou ses sels topiquement acceptables, et de 0,01% à 10% en poids et, plus particulièrement de 0,01 % à 5 % en poids de constituants d'au moins un extrait et/ou une teinture de plantes de la famille des nymphéacées.

7. Composition telle que définie à la revendication 6, qui comprend de 20 % à 40 % en poids de N-palmitoyl proline et de 0,01% à 1% en poids de constituants d'un extrait de nénuphar et, plus particulièrement de fleur de nénuphar.

8. Composition telle que définie à l'une des revendications 1 à 7, qui comprend en outre de 0,1 % à 10 % en poids d'aspartate de magnésium et de potassium et/ou de 0,1% à 10% en poids de gluconate de zinc.

9. Utilisation de la composition telle que définie à l'une des revendications 1 à 8 en cosmétique.

10. Utilisation selon la revendication 9 pour apaiser /ou protéger les peaux sensibles, pour hydrater les peaux sèches, et/ou pour ralentir le vieillissement de la peau et/ou traiter les peaux à tendance acnéique et/ou traiter le cuir chevelu, les méthodes de traitement thérapeutique du corps humain étant exclues.

11. Utilisation selon la revendication 10 comme composition après-solaire, les méthodes de traitement thérapeutique du corps humain étant exclues.

12. Formulation cosmétique susceptible d'être obtenue par dilution du 1/10 jusqu'au 1/20000, de préférence de 1/10 au 1/100, de la composition telle que définie par l'une quelconque des revendications 1 à 8, dans un ou plusieurs excipients cosmétiquement acceptables.

13. Formulation telle que définie à la revendication 12 sous forme d'une émulsion huile dans eau ayant l'aspect d'un lait ayant une viscosité inférieure à 1Pa.s. comprenant comme émulsionnant une composition auto-émulsionnable à base d'alcools gras.

14. Formulation telle que définie à la revendication 12, sous forme d'une crème ou d'un lait apaisant pour traiter les peaux sensibles et particulièrement la peau du visage.

15. Formulation telle que définie à la revendication 12 sous forme d'une formule moussante ou d'un shampooing.

16. Formulation cosmétique comprenant à titre de principe actif de 0,001% à 6% en poids d'au moins un composé de formule (I) et de 0,00005% à 1% en poids de constituants d'un extrait (II) de plantes de la famille des nymphéacés, et comprenant de préférence à titre de principe actif, de 0,1% à 2 % en poids de paimitoyl proline et de 0,0001% à 0,01% en poids de constituants d'au moins un extrait de nénuphar et, plus particulièrement de fleur de nénuphar.

17. Formulation cosmétique telle que définie à la revendication 16 comprenant en outre un ou plusieurs composés à structure lipoaminoacide et de préférence comprenant en outre de 0,1% à 1% en poids d'undécylènoyl glycine et/ou de 0,1% à 1% en poids d'octanoyl glycine.

## Claims

1. Composition, **characterized in that** it comprises as active principle at least one compound of formula (I) : 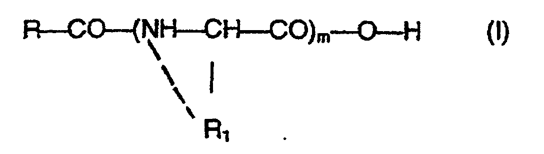 or the topically acceptable salts thereof,
in which R represents the characterizing chain of a saturated or unsaturated, linear or branched fatty acid containing from 3 to 30 carbon atoms, R₁ represents a characterizing chain of an amino acid and m is between 1 and 5, and the constituents of at least one extract and/or of at least one tincture of plants from the Nympheacea family.

2. Composition as defined in Claim 1, for which, in formula (I), the fragment R-CO contains from 7 to 22 carbon atoms and especially represents one of the following radicals: hexanoyl, heptanoyl, octanoyl (capryloyl), decanoyl (caproyl), undecylenoyl, dodecanoyl (lauroyl), tetradecanoyl (myristyl), hexadecanoyl (palmitoyl), octadecanoyl (stearyl), eicosanoyl (arachidoyl), docosanoyl (behenoyl), octadecenoyl (oleyl), eicosenoyl (gadoloyl), docosenoyl (erucyl), octadecadienoyl (linolenoyl).

3. Composition as defined in either of Claims 1 and 2, for which, in formula (I), the fragment R-CO (I) contains from 14 to 18 carbon atoms.

4. Composition as defined in one of Claims 1, 2 and 3, for which, in formula (I), R₁ represents the characterizing chain of proline.

5. Composition as defined in one of Claims 1 to 4, in which the extract or tincture of plants from the Nympheacea family is an extract of water lily and more particularly of water lily flower.

6. Composition as defined in one of Claims 1 to 5, which comprises from 15% to 60% and more particularly from 20% to 40% by weight of at least one compound of formula (I) in which the fragment R-CO contains from 8 to 18 carbon atoms, or its topically acceptable salts, and from 0.01% to 10% by weight and more particularly from 0.01% to 5% by weight of constituents of at least one extract and/or one tincture of plants from the Nympheacea family.

7. Composition as defined in Claim 6, which comprises from 20% to 40% by weight of N-palmitoylproline and from 0.01% to 1% by weight of constituents of an extract of water lily and more particularly of water lily flower.

8. Composition as defined in one of Claims 1 to 7, which also comprises from 0.1% to 10% by weight of magnesium potassium aspartate and/or from 0.1% to 10% by weight of zinc gluconate.

9. Use of the composition as defined in one of Claims 1 to 8, in cosmetics.

10. Use according to Claim 9, for soothing and/or protecting sensitive skin, for moisturizing dry skin and/or for slowing down ageing of the skin and/or treating acne-prone skin and/or treating the scalp, therapeutic methods for treating the human body being excluded.

11. Use according to Claim 10, as an after-sun composition, therapeutic methods for treating the human body being excluded.

12. Cosmetic formulation which may be obtained by dilution from 1/10 to 1/20 000 and preferably from 1/10 to 1/100 of the composition as defined in any one of Claims 1 to 8, in one or more cosmetically acceptable excipients.

13. Formulation as defined in Claim 12, in the form of an oil-in-water emulsion having the appearance of a milk with a viscosity of less than 1 Pa.s, comprising as emulsifier a self-emulsifying composition based on fatty alcohols.

14. Formulation as defined in Claim 12, in the form of a soothing cream or milk for treating sensitive skin and particularly facial skin.

15. Formulation as defined in Claim 12, in the form of a foaming formula or a shampoo.

16. Cosmetic formulation comprising as active principle from 0.001% to 6% by weight of at least one compound of formula (I) and from 0.00005% to 1% by weight of constituents of an extract (II) of plants from the Nympheacea family, and preferably comprising as active principle from 0.1% to 2% by weight of palmitoylproline and from 0.0001% to 0.01% by weight of constituents of at least one extract of water lily and more particularly of water lily flower.

17. Cosmetic formulation as defined in Claim 16, also comprising one or more compounds of lipoamino acid structure and preferably also comprising from 0.1% to 1% by weight of undecylenoylglycine and/or from 0.1% to 1% by weight of octanoylglycine.

## Patentansprüche

1. Zusammensetzung, **dadurch gekennzeichnet, daß** sie als Wirkstoff mindestens eine Verbindung der Formel (I) 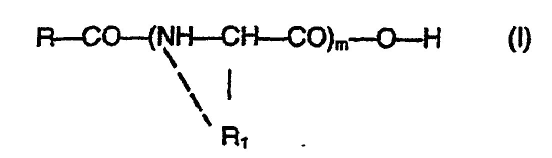 oder ihre topisch unbedenklichen Salze,
worin R die typische Kette einer gesättigten oder ungesättigten geradkettigen oder verzweigten Fettsäure mit 3 bis 30 Kohlenstoffatomen bedeutet, R₁ eine typische Aminosäurekette bedeutet und m 1 bis 5 bedeutet, sowie Bestandteile von mindestens einem Extrakt und/oder mindestens einem Auszug von Pflanzen der Familie der Nymphaeaceae enthält.

2. Zusammensetzung nach Anspruch 1, bei der in Formel (I) der Molekülteil R-CO 7 bis 22 Kohlenstoffatome beinhaltet und insbesondere einen der Reste Hexanoyl, Heptanoyl, Octanoyl (Capryloyl), Decanoyl (Caproyl), Undecylnoyl, Dodecanoyl (Lauroyl), Tetradecanoyl (Myristyl), Hexadecanoyl (Palmitoyl), Octadecanoyl (Stearyl), Eicosanoyl (Arachidoyl), Docosanoyl (Behenoyl), Octadecenoyl (Oleyl), Eicosenoyl (Gadoloyl), Docosenoyl (Erucyl) bzw. Octadecadienoyl (Linolenoyl) bedeutet.

3. Zusammensetzung nach Anspruch 1 oder 2, bei der in Formel (I) der Molekülteil R-CO (I) 14 bis 18 Kohlenstoffatome beinhaltet.

4. Zusammensetzung nach einem der Ansprüche 1, 2 oder 3, bei der in Formel (I) R₁ die für Prolin typische Kette bedeutet.

5. Zusammensetzung nach einem der Ansprüche 1 bis 4, bei der es sich bei dem Extrakt oder Auszug von Pflanzen der Familie der Nymphaeaceae um einen Seerosenextrakt, insbesondere einen Seerosenblütenextrakt, handelt.

6. Zusammensetzung nach einem der Ansprüche 1 bis 5, die 15 bis 60 Gew.-%, insbesondere 20 bis 40 Gew.-%, mindestens einer Verbindung der Formel (I), in der der Molekülteil R-CO 8 bis 18 Kohlenstoffatome enthält, oder ihrer topisch unbedenklichen Salze sowie 0,01 bis 10 Gew.-%, insbesondere 0,01 bis 5 Gew.-%, an Bestandteilen von mindestens einem Extrakt und/oder einem Auszug von Pflanzen der Familie der Nymphaeaceae enthält.

7. Zusammensetzung nach Anspruch 6, die 20 bis 40 Gew.-% N-Palmitoylprolin und 0,01 bis 1 Gew.-% an Bestandteilen eines Seerosenextrakts, insbesondere eines Seerosenblütenextrakts, enthält.

8. Zusammensetzung nach einem der Ansprüche 1 bis 7, die weiterhin 0,1 bis 10 Gew.-% Magnesium- und Kaliumaspartat und/oder 0,1 bis 10 Gew.-% Zinkgluconat enthält.

9. Verwendung der Zusammensetzung nach einem der Ansprüche 1 bis 8 in der Kosmetik.

10. Verwendung nach Anspruch 9 zur Beruhigung und/oder zum Schutz von empfindlicher Haut, zur Hydratisierung von trockener Haut und/oder zur Verzögerung der Hautalterung und/oder zur Behandlung von Haut, die zu Akne neigt, und/oder zur Behandlung der Kopfhaut, mit Ausnahme von Verfahren zur therapeutischen Behandlung des menschlichen Körpers.

11. Verwendung nach Anspruch 10 als After-Sun-Produkt, mit Ausnahme von Verfahren zur therapeutischen Behandlung des menschlichen Körpers.

12. Kosmetikformulierung erhältlich durch 1/10- bis 1/20000-, vorzugsweise 1/10- bis 1/100-Verdünnung der Zusammensetzung nach einem der Ansprüche 1 bis 8 in einem oder mehreren kosmetisch unbedenklichen Grundstoffen.

13. Formulierung nach Anspruch 12 in Form einer Öl-in-Wasser-Emulsion mit milchartigem Aussehen und einer Viskosität unter 1 Pa.s, die als Emulgator eine selbstemulgierbare Zusammensetzung auf Fettalkoholgrundlage enthält.

14. Formulierung nach Anspruch 12 in Form einer beruhigenden Crème oder Milch zur Behandlung von empfindlicher Haut, insbesondere der Gesichtshaut.

15. Formulierung nach Anspruch 12 in Form eines schaumbildenden Produkts oder eines Shampoos.

16. Kosmetikformulierung, die als Wirkstoff 0,001 bis 6 Gew.-% mindestens einer Verbindung der Formel (I) und 0,00005 bis 1 Gew.-% an Bestandteilen eines Extrakts (II) von Pflanzen der Familie der Nymphaeaceae enthält, und die vorzugsweise als Wirkstoff 0,1 bis 2 Gew.-% Palmitoylprolin und 0,0001 bis 0,01 Gew.-% an Bestandteilen von mindestens einem Seerosenextrakt, insbesondere einem Seerosenblütenextrakt, enthält.

17. Kosmetikformulierung nach Anspruch 16, die weiterhin eine oder mehrere Verbindungen mit Lipoaminosäurestruktur und vorzugsweise weiterhin 0,1 bis 1 Gew.-% Undecylenoylglycin und/oder 0,1 bis 1 Gew.-% Octanoylglycin enthält.
